# EUROPEAN PATENT APPLICATION

(11) **EP 2 680 003 A1**
(43) Date of publication of application: **01.01.2014**
(21) Application number: 12382257.9
(22) Date of filing: 28.06.2012
(51) Int. Cl.: G01N 33/574

(54) **Serum biomarker for diagnosing colorectal cancer**

(71) Applicant: Fundació Institut d'Investigació Biomèdica de Bellvitge, 08908 L'Hospitalet de Llobregat - Barcelona (ES); Institut Català D'Oncologia, 08908 l'Hospitalet de Llobregat - Barcelona (ES); Universitat de Barcelona, 08028 Barcelona (ES)
(72) Inventor: Moreno Aguado, Victor Raul, 08970 St. Joan Despi - Barcelona (ES); Crous Bou, Marta, 17300 Blanes - Girona (ES); Solé Acha, Xavier, 08029 Barcelona (ES); Cordero Romera, David, 08908 L´Hospitalet - Barcelona (ES); Berenguer Llergo, Antoni, 08206 Sabadell - Barcelona (ES); Guinó Doménech, Elisabeth, 08018 Barcelona (ES); Sanz Pamplona, Rebeca, 08030 Barcelona (ES)
(74) Representative: ABG Patentes, S.L.

(57) **Abstract**

The present invention relates to an *in vitro* method for the diagnosis of colorectal cancer in a subject using a novel colorectal cancer serum protein biomarker. Said method comprises determining the level of COL10A1 protein in a biofluid. Further, the present invention relates to the assessment of a therapy of colorectal cancer using said biomarker, a method for identifying a therapy useful in the treatment of colorectal cancer using said biomarker, the use of an antibody to said biomarker and kits for all these aspects.

## Description

### FIELD OF THE INVENTION

The present invention relates to the diagnosis of colorectal cancer using a novel colorectal cancer serum protein biomarker. Further, it relates to the assessment of a therapy of colorectal cancer using said biomarker, a method for identifying a therapy useful in the treatment of colorectal cancer using said biomarker, the use of an antibody to said biomarker and kits for all these aspects.

### BACKGROUND OF THE INVENTION

Colorectal cancer (CRC), commonly known as bowel cancer, is the third most common malignancy in the world and represents approximately ten percent of the world's total cancer incidence. It is caused by uncontrolled cell growth (neoplasia), in the colon, rectum, or vermiform appendix. Colorectal cancers start in the lining of the bowel. If left untreated, they can grow into the muscle layers underneath and then through the bowel wall. Most begin as a small growth on the bowel wall: a colorectal polyp or adenoma. These are usually benign, but some develop into cancer over time.

The chance of surviving CRC is closely related to the stage of the disease at diagnosis; the earlier the diagnosis, the greater the likelihood of survival. For example, there is less than a 20% chance of 5-year survival when diagnosed late in the disease timeframe (stage IV), while there is greater than 90% chance of 5-year survival when diagnosed early (stage I). As early diagnosis offers the best chance for a curative treatment, CRC patients would greatly benefit from early detection because of the effectiveness of surgical treatment early on.

The most common and earliest detection procedures available at present for CRC are (i) the fecal occult blood test (FOBT), which is based on the assumption that cancers will bleed, and can therefore be detected in the stool using chemical or immunological assays; significant tumor size must typically exist before fecal blood is detected; (ii) imaging methods such as virtual colonoscopy; and (iii) invasive methods that identify gross abnormalities such as sigmoidoscopy or colonoscopy.

The FOBT is the most widespread clinically useful test used for CRC, and involves a crude test for the peroxidase-like activity of heme in hemoglobin (guayac test) or a specific antibody against hemoglobin (immunologic test). However, this screening technique suffers from a certain number of handicaps: The major drawback is its mediocre sensitivity of approximately 50%, with 20% sensitivity for adenomas (which, if they are large in size, will result in the development of cancer in 1 case out of 10), due to the fact that not all adenomas and CRCs bleed. The test is also not very specific as the appearance of blood in the stools may be related to a non-tumour condition (e.g. ulcerative colitis, hemorrhoids, fistulae, etc.). Thus, a colonoscopy must also be carried out, with the drawbacks described below.

Computerized Tomography Colonography (CTC), or virtual colonoscopy, is a recent non-invasive technique for imaging the colon, with reports varying dramatically on the performance characteristics of the assay (ranging between 39% and 94% specificity), due primarily to technological differences in the patient preparation and the hardware and software used for the analysis. Other limitations of CTC include high false-positive readings, inability to detect flat adenomas, no capacity to remove polyps, repetitive and cumulative radiation doses, and cost.

Invasive methods for identifying gross abnormalities include sigmoidoscopy and colonoscopy. Colonoscopy is usually the preferred method for screening average and increased-risk individuals over the age of 50 who have a history of CRC or prior adenomatous polyps, or other predisposing diseases such as inflammatory bowel disease. Although colonoscopy is still the standard test for the presence or absence of polyps and CRC, it can miss up to 15% of lesions >1 cm in diameter. Complications with colonoscopy can include perforation, hemorrhage, respiratory depression, arrhythmias, and infection. Approximately one in 1,000 patients suffers perforations and three in 1,000 experience hemorrhaging. Between one and three deaths out of 10,000 tests occur as a result of the procedure. Other disadvantages such as the lack of trained personnel, patient discomfort, and high cost and the low level of compliancy (around 2%) for colonoscopy will likely prevent the colonoscopy from becoming a routine CRC screening method for the general population. Most sporadic CRCs are thought to develop from benign adenomas, of which only a small number will ever develop to malignancy. Given that the time period for malignant development from benign adenoma is five to ten years, the detection of adenomas across the general population by colonoscopy would require a gross overtreatment of patients, being both costly and potentially harmful. Colonoscopy is the next test after a positive FOBT, and with a 60% false positive rate, imposes unnecessary risks that require improvement.

Currently, there is no serological screening test or specific diagnostic test which detects early CRC. Warren et al (BMC Med 9 (2011) 133) have recently described Septin 9 methylation as a sensitive and specific blood test for colorectal cancer. Although there is a commercial test it is not routinely used for screening, as this test needs DNA extraction and testing for methylation, which is more difficult and expensive than a serological test.

In the recent years a large amount of so-called CRC specific genes has been reported. The vast majority of the corresponding research papers or patent applications are based on data obtained by analysis of RNA expression patterns in colon cancer tissue versus a different tissue, an adjacent normal tissue or healthy colon tissue. Such approaches may be summarized as differential mRNA display techniques. However, differences on the level of mRNA are not necessarily mirrored by the level of the corresponding proteins. A protein encoded by a rare mRNA may be found in very high amounts and a protein encoded by an abundant mRNA may nonetheless be hard to detect and find at all. This lack of correlation between mRNA-level and protein level is due to reasons like mRNA stability, efficiency of translation, stability of the protein, etc.

There also are recent approaches investigating the differences in protein patterns between different tissues or between healthy and diseased tissue in order to identify candidate marker molecules which might be used in the diagnosis of CRC. Bruenagel et al. (Cancer Research 62 (2002) 2437-2442), have identified seven nuclear matrix proteins which appear to be more abundant in CRC tissue as compared to adjacent normal tissue. Further, WO 2010/061996 A1 discloses several protein biomarkers, some which can be detected in serum of CRC patients.

However, despite candidate protein markers being proposed, to date their clinical/diagnostic utility has not been demonstrated. Ideally, a new diagnostic marker as a single marker should also lead to a progress in diagnostic sensitivity and/or specificity either if used alone or in combination with one or more other markers, compared to currently used diagnostic methods. At present, for example, diagnostic blood tests based on the detection of carcinoembryonic antigen (CEA) are available to assist diagnosis in the field of CRC. However, CEA is mainly used for follow-up and it cannot be used alone for the screening or for the early diagnosis of CRC because its sensitivity and its specificity are insufficient. This is because this marker is expressed by other types of cancer, and in benign pathologies. Despite everything, it is possible to increase sensitivity without losing specificity by combining, with CEA, another tumor marker.

CRC-specific biomarkers in human serum that could provide diagnosis of CRC or of an assessment of CRC-therapy would be extremely beneficial in the management of CRC risk, prevention, and treatment. A test designed to measure these biomarkers would be widely accepted by the general population as it would be minimally invasive and could possibly be used to monitor an individual's susceptibility to disease prior to resorting to, or in combination with, conventional screening methods.

### SUMMARY OF THE INVENTION

In a first aspect, the present invention relates to an *in vitro* method for diagnosing colorectal cancer, comprising determining the level of COL10A1 protein in a biofluid of a subject, wherein an increased COL10A1 protein level is indicative of colorectal cancer.

In a second aspect, the present invention relates to a method for monitoring the effect of a therapy in at least one patient suffering from colorectal cancer, comprising
(i) determining the level of COL10A1 protein in a biofluid of said patient, and
(ii) comparing said level of COL10A1 protein to at least one level of COL10A1 protein in said biofluid of said patient determined at the start of the therapy and/or determined previously during said therapy,
wherein a stagnation and/or a decrease of the level of COL10A1 protein in said patient indicates a positive effect of said therapy.

In a third aspect, the present invention relates to a method for the identification of a useful therapy for colorectal cancer, comprising
(i) determining the level of COL10A1 protein in a biofluid of at least one patient suffering colorectal cancer and which is being treated with a candidate therapy, and
(ii) comparing the level of COL10A1 protein determined in step (i) to at least a reference value for said COL10A1 protein levels wherein a stagnation and/or a decrease of the level of COL10A1 protein in the biofluid with respect to the reference value indicates that the candidate therapy is useful for treating colorectal cancer
wherein said reference value is selected from the group consisting of the COL10A1 protein expression levels in the biofluid of said patient determined at the start of the therapy, the COL10A1 protein expression levels in the biofluid of said patient determined previously during said therapy and the level of COL10A1 protein in the biofluid of at least one control patient not treated with said candidate therapy.

In a fourth aspect, the present invention relates to the use of a COL10A1 protein antibody for the diagnosis of colorectal cancer, for monitoring the effect of a therapy in a patient suffering from colorectal cancer or for the identification of a therapy for colorectal cancer.

In a fifth aspect, the present invention relates to a kit comprising means for carrying out one or more methods of any of the foregoing aspects.

### BRIEF DESCRIPTION OF THE FIGURES

**Figure 1****:** COL10A1 RNA expression levels in colon tumour (T, right sample, n=100), normal mucosa of patients with cancer (N, middle sample, n=100) and normal mucosa of healthy individuals (M, left sample, n=50).
**Figure 2****:** COL10A1 serum protein concentration in patients with CRC (case, n=16) and healthy individuals (control, n=16).
**Figure 3****:** COL11A1 serum concentration values in patients with CRC and healthy individuals by duplicates (16 duplicated cases, N=32)
**Figure 4****:** COL10A1 serum concentration values by tumor cancer stage at cancer diagnosis.
**Figure 5****:** ROC curve of serum COL10A1.
**Figure 6****:** Prognostic significance value of COL10A1 RNA expression levels in colon cancer patients (N=100).

### DETAILED DESCRIPTION OF THE INVENTION

The present inventors have discovered a novel tumor marker, namely collagen X type alpha 1 (COL10A1), which is released by CRC tumors out of the cancerous tissues and is characteristic of these tumors, such that it can be detected in biofluids remote from the tumors. COL10A1 is one of 28 types of collagen, which have been identified so far. The different collagen types vary significantly in structure, function, site of expression and relevance for disease. COL10A1 is a short chain collagen expressed by hypertrophic chondrocytes during endochondral ossification and is implicated in diseases such as, for example, Schmid metaphyseal dysplasia. However, it was heretofore unknown that COL10A1 can be used as a protein marker in non-cancer tissue such as biofluids to diagnose CRC. Furthermore, the inventors surprisingly found that COL10A1 has a sensitivity and specificity for the diagnosis of CRC, which exceeds that of conventional methods as well as that of other biomarkers for CRC diagnosis.

### Diagnostic method of the invention

In a first aspect, the present invention relates to an *in vitro* method for diagnosing colorectal cancer, comprising determining the level of COL10A1 protein in a biofluid of a subject, wherein an increased COL10A1 protein level is indicative of colorectal cancer.

The term "diagnosis" as used herein refers both to the process of attempting to determine and/or identify a possible disease in a subject, i.e. the diagnostic procedure, and to the opinion reached by this process, i.e. the diagnostic opinion. As such, it can also be regarded as an attempt at classification of an individual's condition into separate and distinct categories that allow medical decisions about treatment and prognosis to be made.

The term "colorectal cancer" (CRC) is used in the broadest sense and refers to (1) all stages and all forms of cancer arising from epithelial cells of the large intestine and/or rectum and/or (2) all stages and all forms of cancer affecting the lining of the large intestine and/or rectum. The term "colorectal cancer", as used herein, also includes adenomas. In the staging systems used for classification of colorectal cancer, the colon and rectum are treated as one organ.

The term "subject", as used herein, refers to all animals classified as mammals and includes, but is not restricted to, domestic and farm animals, primates and humans, e.g., human beings, non-human primates, cows, horses, pigs, sheep, goats, dogs, cats, or rodents. Preferably, the patient is a male or female human of any age or race. A subject may or may not have or suffer from CRC. In other words, it is not known whether the subject has or suffers from CRC.

In a preferred embodiment of the invention, said colorectal cancer (CRC) is colon cancer, rectal cancer and/or vermiform appendix cancer. In another embodiment of the invention, said colorectal cancer is stage 0, stage I, stage II, stage III and/or stage IV colorectal cancer. The stages of CRC referred to herein correspond to the American Joint Committee on Cancer (AJCC) CRC staging, although other staging methods, such as Dukes and Astler-Coller staging, can equally be used.

"Stage 0" refers to the cancer in the earliest stage. It has not grown beyond the inner layer (mucosa) of the colon or rectum. This stage is also known as *carcinoma in situ* or *intramucosal carcinoma.*

"Stage I" refers to the cancer having grown through the muscularis mucosa into the submucosa or also having grown into the muscularis propria. It has not spread to nearby lymph nodes or distant sites.

"Stage II" including sub-stages A, B and C refers to the cancer (A) grown into the outermost layers of the colon or rectum but has not gone through them; it has not reached nearby organs and it has not yet spread to the nearby lymph nodes or distant sites; (B) grown through the wall of the colon or rectum but has not grown into other nearby tissues or organs; it has not yet spread to the nearby lymph nodes or distant sites; and/or (C) grown through the wall of the colon or rectum and is attached to or has grown into other nearby tissues or organs; it has not yet spread to the nearby lymph nodes or distant sites.

"Stage III" including sub-stages A, B and C refers to the following: (A) At least one of the following applies: (i) The cancer has grown through the mucosa into the submucosa and it may also have grown into the muscularis propria. It has spread to 1 to 3 nearby lymph nodes or into areas of fat near the lymph nodes but not the nodes themselves. It has not spread to distant sites. (ii) The cancer has grown through the mucosa into the submucosa. It has spread to 4 to 6 nearby lymph nodes. It has not spread to distant sites. (B) At least one of the following applies: (i) The cancer has grown into the outermost layers of the colon or rectum or through the visceral peritoneum but has not reached nearby organs. It has spread to 1 to 3 nearby lymph nodes or into areas of fat near the lymph nodes but not the nodes themselves. It has not spread to distant sites. (ii) The cancer has grown into the muscularis propria or into the outermost layers of the colon or rectum. It has spread to 4 to 6 nearby lymph nodes. It has not spread to distant sites. (iii) The cancer has grown through the mucosa into the submucosa or it may also have grown into the muscularis propria. It has spread to 7 or more nearby lymph nodes. It has not spread to distant sites. (C) At least one of the following applies: (i) The cancer has grown through the wall of the colon or rectum (including the visceral peritoneum) but has not reached nearby organs. It has spread to 4 to 6 nearby lymph nodes. It has not spread to distant sites. (ii) The cancer has grown into the outermost layers of the colon or rectum or through the visceral peritoneum but has not reached nearby organs. It has spread to 7 or more nearby lymph nodes. It has not spread to distant sites. (iii) The cancer has grown through the wall of the colon or rectum and is attached to or has grown into other nearby tissues or organs. It has spread to at least one nearby lymph node or into areas of fat near the lymph nodes. It has not spread to distant sites.

"Stage IV" including sub-stages A and B refers to the following: (A) The cancer may or may not have grown through the wall of the colon or rectum, and it may or may not have spread to nearby lymph nodes. It has spread to 1 distant organ (such as the liver or lung) or set of lymph nodes. (B) The cancer may or may not have grown through the wall of the colon or rectum, and it may or may not have spread to nearby lymph nodes. It has spread to more than 1 distant organ (such as the liver or lung) or set of lymph nodes, or it has spread to distant parts of the peritoneum (the lining of the abdominal cavity).

The term "COL10A1" or "COL10A1 protein", as used herein, refers to a protein that is encoded by the human *COL10A1* gene or orthologs thereof. In a preferred embodiment of the invention, it is human COL10A1 (RefSeq NP_000484 in the NCBI database, release of Oct 22, 2011). In a more preferred embodiment, the COL10A1 lacks the signal sequences (amino acids 1 to 18 in the polypeptide shown in the NCBI database under accession number NP_000484). Within the scope of this term, however, are also variants of this protein, wherein said variants have a sequence which is at least 85%, at least 90%, at least 95%, at least 98% or preferably at least 99% identical to human COL10A1 or orthologs thereof. Preferred variants are naturally occuring variants, especially variants which are correlated with a predisposition to CRC. Moreover, the term "COL10A1" also refers to fragments of the COL10A1 that might appear in the biofluid, the concentration of which in the biofluid being as informative with respect to the presence of CRC as the full-length COL10A1. Suitable fragments of COL10A1 that may appear in a biofluid and suitable for use in the present invention include, without limitation, fragments of at least 50 amino acids, at least 100 amino acids, at least 150 amino acids, at least 200 amino acids, at least 250 amino acids, at least 300 amino acids, at least 350 amino acids, at least 400 amino acids, at least 450 amino acids, at least 450 amino acids, at least 500 amino acids, at least 550 amino acids, at least 600 amino acids, at least 650 amino acids.

The levels of COL10A1 protein can be determined by a plethora of protein measurement techniques known and sufficiently described in the art. In a preferred embodiment, the level of COL10A1 protein is determined employing a protein measurement technique selected from the group consisting of ELISA, Western blotting dot blotting, immunochromatography, Luminex assay, mass spectrometry, MALDI-TOF, SELDI-TOF or other proteomic techiques and combinations thereof.

Examples of ELISA include direct ELISA using a labeled antibody recognizing an antigen immobilized on a solid support; indirect ELISA using a labeled antibody recognizing a capture antibody forming complexes with an antigen immobilized on a solid support; direct sandwich ELISA using a labeled antibody recognizing an antigen bound to a antibody immobilized on a solid support; and indirect sandwich ELISA, in which a captured antigen bound to an antibody immobilized on a solid support is detected by first adding an antigen-specific antibody, and then a secondary labeled antibody which binds the antigen-specific antibody. More preferably, the protein expression levels are detected by sandwich ELISA, where a sample reacts with an antibody immobilized on a solid support, and the resulting antigen-antibody complexes are detected by adding a labeled antibody specific for the antigen, followed by enzymatic development, or by first adding an antigen-specific antibody and then a secondary labeled antibody which binds to the antigen-specific antibody, followed by enzymatic development. Information necessary for the diagnosis of CRC can be provided by measuring the degree of complex formation of a CRC biomarker protein and an antibody thereto.

Further, the measurement of protein expression levels is preferably achieved using Western blotting using one or more antibodies to the CRC makers. Also, the measurement of protein expression levels is preferably performed by immunochromatography. In addition, the measurement of protein expression levels can be carried out with a Luminex assay.

The antibody to be used with the present invention may be a monoclonal, polyclonal or recombinant antibody, which has high specificity and affinity to each marker protein and rarely has or does not have cross-reactivity with other proteins.

The term "biofluid" is a biological fluid and refers to an aqueous fluid of biological origin. It may be obtained from any location (such as blood, plasma, serum, urine, bile, cerebrospinal fluid, aqueous or vitreous humor, or any bodily secretion).

In a preferred embodiment of the invention, the biofluid is blood, plasma or serum. In a more preferred embodiment, it is serum.

The subject of the method according to the first aspect is an animal, preferably a mammal and most preferably human. The subject may or may not have CRC. In a preferred embodiment, it is not known whether the subject has CRC. Further, the subject may be healthy or not with respect to other diseases. In one embodiment, the subject is a subject to which at least one (or at least 3, or at least 5) CRC risk factor applies. "CRC risk factors" are any non-modifiable or environmental CRC risk factors. In a preferred embodiment, non-modifiable CRC risk factors are selected from the group consisting of high age (e.g., at least 40 years, at least 50 years, at least 60 years or at least 70 years), personal history of adenomatous polyps, personal history of inflammatory bowel disease, family history of colorectal cancer or adenomatous polyps and inherited genetic risk. In another preferred embodiment, environmental CRC risk factors are selected from the group consisting of CRC predisposing nutritional practices, little physical activity, obesity, cigarette smoking and heavy alcohol consumption. More details on CRC risk factors are provided in Haggar and Boushey et al., Clin Colon Rectal Surg. 2009 November; 22(4): 191-197.

A COL10A1 protein level is considered increased if the level is increased compared to a reference that is indicative of colorectal cancer by at least 5%, by at least 10%, by at least 15%, by at least 20%, by at least 25%, by at least 30%, by at least 35%, by at least 40%, by at least 45%, by at least 50%, by at least 55%, by at least 60%, by at least 65%, by at least 70%, by at least 75%, by at least 80%, by at least 85%, by at least 90%, by at least 95%, by at least 100%, by at least 110%, by at least 120%, by at least 130%, by at least 140%, or by at least 150%.

In a preferred embodiment of the invention, an increase of at least 5%, at least 10%, at least 15%, at least 20%, or at least 25%, or at least 30%, or at least 35%, or at least 40%, or at least 45%, or at least 50% compared to a reference that is indicative of colorectal cancer. Said reference value can be any statistical value derived from the COL10A1 levels in a biofluid derived from subjects of a group of subjects not having colorectal cancer and, preferably, not having a history of colorectal cancer. In a preferred embodiment, said reference is the mean or median level of COL10A1 protein in a biofluid in a group of subjects not having colorectal cancer. Said group of subjects comprises at least 3, at least 5, at least 10, at least 15, at least 20, at least 30 or at least 50 subjects not having colorectal cancer.

As indicated above, the method of the first aspect is superior to conventional methods for CRC diagnosis as well as methods using other biomarkers for CRC diagnosis with respect to established evaluation means of diagnostic methods, such as sensitivity or specificity.

In a preferred embodiment, the method of the first aspect is characterised by having a sensitivity of at least 60%, at least 65%, at least 70%, at least 75%, at least 80%, at least 85%, at least 90%, or at least 93%. The term "sensitivity" as referred to herein relates to the method's ability to identify positive results. It measures the proportion of actual positives which are correctly identified as such, i.e. true positives. It is calculated by dividing the number of true positives by the sum of true positives and false negatives.

In another preferred embodiment, the method of the first aspect is characterised by having a specificity of at least 55%, at least 60%, at least 65%, at least 70%, at least 75%, at least 80%, at least 85%, or at least 88%. The term "specificity" as referred to herein relates to the method's ability to identify negative results. It measures the proportion of negatives which are correctly identified, i.e. true negatives. It is calculated by dividing the number of true negatives by the sum of true negatives and false positives.

In another preferred embodiment, the method of the first aspect is characterised by having a positive predictive value of at least 55%, at least 60%, at least 65%, at least 70%, at least 75%, at least 80%, at least 85%, or at least 87%. The term "positive predictive value", as referred to herein, also known as precision rate, is the proportion of subjects with positive test results who are correctly diagnosed. It is calculated by dividing the number of true positives by the sum of true positives and false positives.

In another preferred embodiment, the method of the first aspect is characterised by having a negative predictive value of at least 60%, at least 65%, at least 70%, at least 75%, at least 80%, at least 85%, at least 90%, or at least 93%. The term "negative predictive value", as referred to herein, is the proportion of subjects with a negative test result who are correctly diagnosed. It is calculated by dividing the number of true negatives by the sum of true negatives and false negatives.

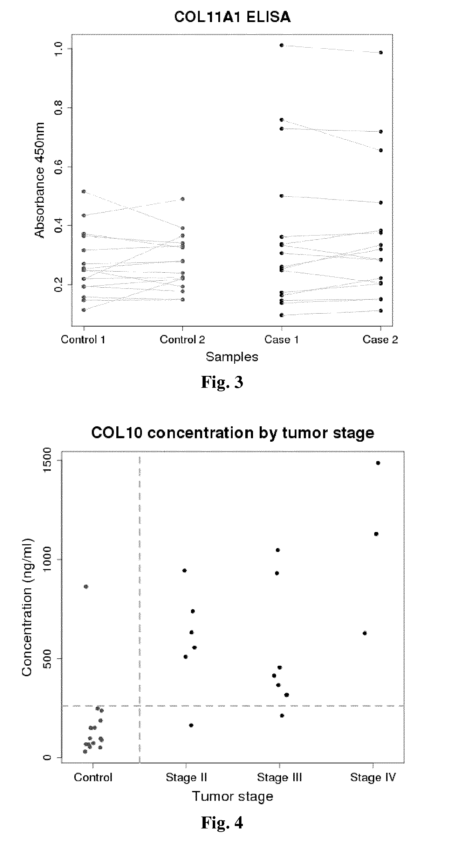

In another preferred embodiment, the method of the first aspect is characterised by having an area under curve (AUC) of the receiver operator characteristic (ROC) curve of at least 0.75, at least 0.80, at least 0.85, at least 0.90 or at least 0.93. A ROC curve is a graphical plot of the sensitivity, or true positive rate, vs. false positive rate (1 - specificity or 1 - true negative rate), for a binary classifier system as its discrimination threshold is varied. The ROC can also be represented equivalently by plotting the fraction of true positives out of the positives (TPR = true positive rate) vs. the fraction of false positives out of the negatives (FPR = false positive rate). The accuracy of a diagnostic test depends on how well the test separates the group being tested into those with and without the disease in question. Accuracy is measured by the area under the ROC curve. The area under ROC curve specifies the probability that, when one draws one positive and one negative example at random, the decision function assigns a higher value to the positive than to the negative example. An area of 1 represents a perfect test; an area of 0.5 represents a worthless test. A rough guide for classifying the accuracy of a diagnostic test is the traditional academic point system: 0.90-1= excellent (A), 0.80-0.90 = good (B), 0.70-0.80 = fair (C), 0.60-0.70 = poor (D), 0.50-0.60 = fail (F). Accordingly, the diagnostic test of the invention would be classified as excellent with a AUC of 0.938 (see Figure 4). Other parameters suitable to assess the quality of the biomarker include, without limitation, sensibility and specificity, predictive positive value and negative predictive value, accuracy and precision.

In a preferred embodiment of the method of the first aspect, said method further comprises the determination of one or more biomarkers selected from the group consisting of CEA, TCN, SULT2B1, ALDOB, COL11A1, PI3, CCL20, MTHFD1L, IL-1b, SRPX2, SLCO4A1, TESC, and/or IL-23a. In a still more preferred embodiment, an increase in the levels or one or more of said CEA, TCN, SULT2B1, ALDOB, COL11A1, PI3, CCL20, MTHFD1L, IL-1b, SRPX2, SLC04A1, TESC biomarkers is indicative of colorectal cancer.

CEA (carcinoembryonic antigen) protein is a glycoprotein involved in cell adhesion. In a preferred embodiment, the term "CEA" relates to human CEA (NCBI Reference Sequence: NP_004354.2).

TCN (transcobalamin-1) protein is a glycoprotein produced by the salivary glands of the oral cavity, in response to ingestion of food. In a preferred embodiment, the term "TCN" relates to human TCN (NCBI Reference Sequence: NP_001053.2).

SULT2B1 (Sulfotransferase family cytosolic 2B member 1) protein catalyzes the sulfate conjugation of hormones, neurotransmitters, drugs, and xenobiotic chemical compounds. In a preferred embodiment, the term "SULT2B1" relates to human SULT2B1 (NCBI Reference Sequence: NP_004596.2).

ALDOB (Aldolase B) protein also known as fructose-bisphosphate aldolase B or liver-type aldolase is one of three isoenzymes (A, B, and C) of the class I fructose 1,6-bisphosphate aldolase enzyme (EC 4.1.2.13), and plays a key role in both glycolysis and gluconeogenesis. In a preferred embodiment, the term "ALDOB" relates to human ALDOB (NCBI Reference Sequence: NP_000026.2).

COL11A1 (Collagen alpha-1(XI) chain) protein is a minor fibrillar collagen. In a preferred embodiment, the term "COL11A1" relates to human COL11A1 (GenBank: AAI17698.1).

PI3 (peptidase inhibitor 3) protein contains a WAP-type four-disulfide core (WFDC) domain. In a preferred embodiment, the term "PI3" relates to human PI3 (NCBI Reference Sequence: NP_002629.1).

CCL20 (Chemokine (C-C motif) ligand 20) protein is a small cytokine belonging to the CC chemokine family and it is strongly chemotactic for lymphocytes and weakly attracts neutrophils. In a preferred embodiment, the term "CCL20" relates to human CCL20 (NCBI Reference Sequence: NP_001123518.1).

MTHFD1L (methylenetetrahydrofolate dehydrogenase (NADP+ dependent) 1-like) protein is an enzyme involved in THF synthesis in mitochondria. In a preferred embodiment, the term "MTHFD1L" relates to human MTHFD1L (GenBank: CAI95775.1).

IL-1b (Interleukin-1 beta) protein is an important mediator of the inflammatory response, and is involved in a variety of cellular activities, including cell proliferation, differentiation, and apoptosis. In a preferred embodiment, the term "IL-1b" relates to human IL-1b (NCBI Reference Sequence: NP_000567.1).

SRPX2 (Sushi repeat-containing protein SRPX2) protein is a putative peroxiredoxin. In a preferred embodiment, the term "SRPX2" relates to human SRPX2 (NCBI Reference Sequence: NP_055282.1).

SLC04A1 (solute carrier organic anion transporter family, mernber 4A1) protein has the function of mediating the Na+- independent transport of organic anions such as thyroid hormone T3. In a preferred embodiment, the term "SLC04A1" relates to human SLC04A1 (NCBI Reference Sequence: NP_057438.3).

TESC (tescalcin) protein is an EF-hand calcium binding protein that regulates the Na+/H+ exchanger 1. In a preferred embodiment, the term "TESC" relates to human TESC (GenBank: AAL35615.1).

IL-23a (Interleukin-23 subunit alpha) protein is an important part of the inflammatory response against infection. It promotes upregulation of the matrix metalloprotease MMP9, increases angiogenesis and reduces CD8+ T-cell infiltration.

In a preferred embodiment, the term "IL-23a" relates to human IL-23a (NCBI Reference Sequence: NP_057668.1).

In another preferred embodiment of the method of the first aspect, said method further comprises one or more diagnostic means selected from the group consisting of digital rectal exam, fecal occult blood test, sigmoidoscopy, colonoscopy, colonography, imaging test (e.g. X-ray, ultrasound, CT or MRI scan).

In another preferred embodiment of the first method of the invention, the method further comprises the determination of the, wherein the presence of blood in faeces is indicative of the presence of colorectal cancer

The expression "presence of blood in faeces". Suitable means for determining the presence of blood in faeces include, without limitation, the fecal occult blood test (FOBT) also called stool gaiac or hemoccult test as described in US patents 3,996,006, 4,333,734, 4,071,317. This test uses chemical or immunological indicators to detect the presence of blood on stool samples.

### Method for monitoring the effect of a therapy

In a second aspect, the present invention relates to a method for monitoring the effect of a therapy in at least one patient suffering from colorectal cancer, comprising
(i) determining the level of COL10A1 protein in a biofluid of said patient, and
(ii) comparing said level of COL10A1 protein to at least one level of COL10A1 protein in said biofluid of said patient determined at the start of the therapy and/or determined previously during said therapy,
wherein a stagnation and/or a decrease of the level of COL10A1 protein in said patient indicates a positive effect of said therapy.

The term "therapy" or "treatment", as used herein, refers to the attempted remediation of a health problem, usually following a diagnosis. As such, it is not necessarily a cure, i.e. a complete reversion of a disease. Said therapy may or may not be known to have a positive effect on CRC or, in other words, to be useful to treat CRC. Therapies include, without limitation, surgery, radiotherapy, chemotherapy, or targeted therapies (e.g. using antibodies) and the choice of a therapy or a combination of therapies depends, for example, on the stage of the cancer, whether the cancer has recurred and the patient's general health.

The term "surgery", as used herein, means any therapeutic procedure that involves methodical action of the hand or of the hand with an instrument, on the body of a human or other mammal, to produce a curative or remedial.

The term "radiotherapy" refers to multiple types of radiation therapy including internal and external radiation therapies or radioimmunotherapy, and the use of various types of radiations including X-rays, gamma rays, alpha particles, beta particles, photons, electrons, neutrons, radioisotopes, and other forms of ionizing radiations. In a preferred embodiment, the therapy is neoadjuvant or adjuvant chemotherapy. The term "neoadjuvant therapy", as used herein, refers to any type of treatment of cancer given prior to surgical resection of the primary tumor, in a patient affected

As used herein, the term "chemotherapy" refers to the use of a chemical drug or a combination thereof for the treatment of cancer, tumors or malign neoplasia, including both cytotoxic or cytostatic drugs. Examples of chemotherapy agents which may be in accordance to the present invention include: alkylating agents (for example mechlorethamine, chlorambucil, cyclophosphamide, ifosfamide, streptozocin, carmustine, lomustine, melphalan, busulfan, dacarbazine, temozolomide, thiotepa or altretamine); platinum drugs (for example cisplatin, carboplatin or oxaliplatin); antimetabolite drugs (for example 5-fluorouracil, capecitabine, 6-mercaptopurine, methotrexate, gemcitabine, cytarabine, fludarabine or pemetrexed); anti-tumor antibiotics (for example daunorubicin, doxorubicin, epirubicin, idarubicin, actinomycin-D, bleomycin, mitomycin-C or mitoxantrone); mitotic inhibitors (for example paclitaxel, docetaxel, ixabepilone, vinblastine, vincristine, vinorelbine, vindesine or estramustine); and topoisomerase inhibitors (for example etoposide, teniposide, topotecan, irinotecan, diflomotecan or elomotecan).

The term "targeted therapy" refers to a type of medication that blocks the growth of or kills cancer cells by interfering with specific targeted molecules in or on tumor cells rather than by simply interfering with rapidly dividing cells (e.g. with traditional chemotherapy). The main agent types used for targeted therapies are small molecules and antibodies.

The term "patient", as used herein, refers to a subject as defined above which suffers and is known to suffer from CRC.

"Start of said therapy" means a time point just before the therapy is applied, preferably immediately before, e.g. before and on the same or the previous day. The term "previously", as used herein, refers to any prior time point. Preferably though it means at least 1 day, at least 2 days, at least 4 days, at least 5 days, at least 6 days, at least 7 days, at least 10 days, at least 2 weeks, at least 3 weeks, at least 4 weeks, at least 6 weeks, at least 2 months or at least 3 months before. It is emphasised that the level of step (i) can be compared to more than one level of step (ii), wherein the more than one levels of step (ii) are preferably determined at different time points, e.g. any of the time points recited above.

"Stagnation" preferably means a level within a 3%, a 5%, an 8%, a 10%, a 15%, or a 20% range of a level determined before, e.g. previously as defined above. In a preferred embodiment, this "level determined before" is the value determined at the start of the therapy.

"Decrease" preferably refers to a level at least 3%, at least 5%, at least 8%, at least 10%, at least 15%, or at least 20% lower than a level determined before, e.g. previously as defined above. Similarly, decrease can refer to a level at least 5%, at least 10%, at least 15%, at least 20%, at least 25%, at least 30%, at least 35%, at least 40%, at least 45%, at least 50%, at least 55%, at least 60%, at least 65%, at least 70%, at least 75%, at least 80%, at least 85%, at least 90%, at least 95%, or at least 100% (i.e., absent) lower than a level determined before, e.g. previously as defined above. In a preferred embodiment, this "level determined before" is the value determined at the start of the therapy.

A "positive effect of a therapy" means that said therapy slows down disease progression, prevents disease progression, reduces or eliminates tumour burden (i.e. size and/or number of tumours), and/or cures the patient. It may also mean an increase in the duration of Recurrence-Free interval (RFI), an increase in the time of survival or Overall Survival (OS), an increase in the time of Disease-Free Survival (DFS), an increase in the duration of Distant Recurrence-Free Interval (DRFI), and/or the like.

In a preferred embodiment of the method of the second aspect, a decrease of the level of COL10A1 protein in a stage 0, stage I, stage II, stage III or stage IV CRC patient indicates a positive effect of said therapy. In another preferred embodiment, a stagnation of the level of COL10A1 protein in a stage III CRC patient indicates a positive effect of said therapy.

Other terms used to define the method of the second aspect have the meaning as defined with respect to the method of the first aspect. Equally, embodiments described to further characterize the method of the first aspect also further characterize the method of the second aspect, if applicable.

### Methods for the identification of a CRC therapy

In a third aspect, the present invention relates to a method for the identification of therapy for colorectal cancer, comprising
(i) determining the level of COL10A1 protein in a biofluid of at least one patient suffering colorectal cancer and which is being treated with a candidate therapy, and
(iia) comparing the level of COL10A1 protein determined in step (i) to at least a reference value for said COL10A1 protein levels wherein a stagnation and/or a decrease of the level of COL10A1 protein in the biofluid with respect to the reference value indicates that the candidate therapy is useful for treating colorectal cancer
wherein said reference value is selected from the group consisting of the COL10A1 protein expression levels in the biofluid of said patient determined at the start of the therapy, the COL10A1 protein expression levels in the biofluid of said patient determined previously during said therapy and the level of COL10A1 protein in the biofluid of at least one control patient not treated with said candidate therapy.

A "useful therapy" is a therapy which has a positive effect on colorectal cancer in at least one, preferably more than one patient suffering from CRC, but not necessarily in all patients suffering from CRC. A "candidate therapy" according to the method of the second aspect is a therapy which may be, but is not yet known to be a generally useful therapy.

The method of the third aspect may also be carried out for the identification of a useful therapy for colorectal cancer in a subgroup of CRC patients. Such a "subgroup of CRC patients" is a group of patients which is unified by a characteristic relevant to CRC which other CRC patients do not share with this subgroup. Such a characteristic may be the genetic background (e.g. that of identical twins, clones, family members, members of the same race etc.), a specific gene mutation, preferably a mutation increasing the risk for CRC, one or more CRC risk factors etc.

Other terms used to define the method of the third aspect have the meaning as defined with respect to the methods of the first and the second aspect. Equally, embodiments described to further characterize the methods of the first and second aspect also further characterize the method of the third aspect, if applicable.

### Kit of the invention

In a fifth aspect, the present invention relates to a kit comprising means for carrying out one or more methods of any of the foregoing aspects.

As used herein, the term "kit" is used in reference to a combination of articles that facilitate a process, method, use or application. These kits provide the materials necessary for carrying out the application described in the present invention.

In some embodiments, the reagents adequate for the determination of the expression levels of one or more proteins comprise at least 50%, at least 55% at least 60%, at least 65%, at least 70%, at least 75%, at least 80%, at least 90%, at least 95%, at least 96%, at least 97%, at least 98% or at least 99% of the total amount of reagents forming the kit. In a preferred embodiment, said "reagents" are antibodies which bind to said proteins.

In a preferred embodiment, the reagents adequate for the determination of the expression levels of one or more proteins are antibodies.

The term "antibody", as used herein, refers to an immunoglobulin/protein that recognizes a unique part of a targeted protein and could bind it.

The term "antibody" as used to describe this and other aspects of the invention may be a natural polyclonal or monoclonal antibody or a non-natural antibody, e.g. a single domain antibody, a single chain variable fragment antibody, a microantibody etc. Methods for producing such antibodies are well known in the art.

Other terms used to define the use of the fourth aspect have the meaning as defined with respect to the methods of the foregoing aspects. Equally, embodiments described to further characterize the methods of foregoing aspects also further characterize the use of the fourth aspect, if applicable.

In a more preferred embodiment, said kit comprises a COL10A1 antibody and one or more antibodies selected from the group consisting of CEA, TCN, SULT2B1, ALDOB, COL11A1, PI3, CCL20, MTHFD1L, IL-1b, SRPX2, SLCO4A1, TESC, and/or IL-23a antibody.

Preferably, said kit contains up to 2, up to 3, up to 4, up to 5, up to 6, up to 7, up to 8, up to 9, up to 10, up to 11, up to 12, up to 13 or up to 14 different antibodies, i.e. antibodies directed to said number(s) of different proteins.

In some embodiments, the antibodies specific for the biomarkers of the invention will be labeled with a detectable marker (such as a fluorescent dye or a detectable enzyme), or be modified to facilitate detection (e.g., biotinylated to allow for detection with a avidin- or streptavidin-based detection system). In other embodiments, the biomarker-specific reagent will not be directly labeled or modified.

Certain kits of the invention will also include one or more agents for detection of bound reagent. As will be apparent to those of skill in the art, the identity of the detection agents will depend on the type of AD biomarker-specific reagent(s) included in the kit, and the intended detection system. Detection agents include antibodies specific for the AD biomarker-specific reagent (e.g., secondary antibodies), primers for amplification of an AD biomarker-specific reagent that is nucleotide based (e.g., aptamer) or of a nucleotide 'tag' attached to the AD biomarker-specific reagent, avidin-or streptavidin-conjugates for detection of biotin-modified AD biomarker-specific reagent(s), and the like. Detection systems are well known in the art, and need not be further described here.

In certain embodiments, kits for use in the methods disclosed herein include the reagents in the form of an array. The array includes at least two different reagents reagents adequate for the determination of the expression levels of one or more proteins (each reagent specific for a different protein) bound to a substrate in a predetermined pattern (e.g., a grid). Accordingly, the present invention provides arrays comprising reagents adequate for the determination of the expression levels of one or more proteins include, but not limited to COL10A1 antibody and one or more antibodies selected from the group consisting of CEA, TCN, SULT2B1, ALDOB, COL11A1, PI3, CCL20, MTHFD1L, IL-1b, SRPX2, SLCO4A1, TESC, and/or IL-23a antibody. The localization of the different reagents (the "capture reagents") allows measurement of levels of a number of different proteins in the same reaction. Kits including the reagents in array form are commonly in a sandwich format, so such kits may also comprise detection reagents. Normally, the kit will include different detection reagents, each detection reagent specific to a different antibody. The detection reagents in such embodiments are normally reagents specific for the same proteins as the reagents bound to the substrate (although the detection reagents typically bind to a different portion or site on the protein than the substrate-bound reagents), and are generally affinity-type detection reagents. As with detection reagents for any other format assay, the detection reagents may be modified with a detectable moiety, modified to allow binding of a separate detectable moiety, or be unmodified. Array-type kits including detection reagents that are either unmodified or modified to allow binding of a separate detectable moiety may also contain additional detectable moieties (e.g., detectable moieties which bind to the detection reagent, such as labeled antibodies which bind unmodified detection reagents or streptavidin modified with a detectable moiety for detecting biotin-modified detection reagents).

Kits comprising a single reagent will generally have the reagent enclosed in a container (e.g., a vial, ampoule, or other suitable storage container), although kits including the reagent bound to a substrate (e.g., an inner surface of an assay reaction vessel) are also contemplated. Likewise, kits including more than one reagent may also have the reagents in containers (separately or in a mixture) or may have the reagents bound to a substrate.

The kit may comprise, in addition, a packaging which allows maintaining the reagents within determined limits. Suitable materials for preparing such packings include glass, plastic (polyethylene, polypropylene, polycarbonate and the like), bottles, vials, paper, sachets and the like.

The kit of the invention can additionally contain instructions for using the components contained therein, in particularly those mentioned with respect to the foregoing aspects of the invention (e.g., antibodies or ELISA tools). The instructions relating to the use of the kit for carrying out the invention generally describe how the contents of the kit are used to carry out the methods of the invention. Instructions may include information as sample requirements (e.g., form, pre-assay processing, and size), steps necessary to measure the biomarker(s), and interpretation of results.

Said instructions can be found in the form of printed material or in the form of an electronic support which can store instructions such that they can be read by a subject, such as electronic storage media (magnetic disks, tapes and the like), optical media (CD-ROM, DVD) and the like. The media can additionally or alternatively contain Internet websites providing said instructions.

The following examples are merely illustrative and do not limit the scope of the invention in any way.

### EXAMPLES

### MATERIALS & METHODS

### Samples and gene expression assessment for the Discovery Series

For the discovery of candidate biomarkers a homogeneous and well-characterized set of samples was used: 100 samples of paired tumor-mucosa tissue of patients diagnosed with stage II, microsatellite stable colon cancer. All patients were surgically treated and did not receive neoadjuvant chemotherapy. In addition, samples of colon mucosa from 50 healthy donors, obtained during colonoscopy, were studied as control group.

For all these samples, mRNA expression was analyzed with the Affymetrix Human Genome U219 microarray. The array is comprised of more than 530,000 probes covering more than 36,000 transcripts and variants, which represent more than 20,000 genes mapped through UniGene or via RefSeq annotation. Specifically, two probes of COL10A1 gene were included in the array to assess COL10A1 RNA expression levels in colon tumour, normal mucosa of patients with cancer and normal mucosa of healthy individuals.

### Technical and biological validation

Quantitative real time PCR (qPCR) was used to validate with a different technique the COL10A1 expression measurements performed in the same samples of colon tumour and normal mucosa of patients with cancer and normal mucosa of healthy individuals. For the biological validation, an independent series of patients with colorectal cancer was also analyzed using qPCR technique. For this, 80 cases of colorectal cancer in stages I-IV, without excluding the MSI cases and 30 healthy controls were analyzed.

### Samples and protein serum levels assessment for the proof of concept

Tumor tissue or colonic mucosa is not useful for a screening test. Therefore, to become a good biomarker candidate, COL10A1 protein was measured in blood. A commercial ELISA detection kit was used to measure protein levels in serum samples from colon cancer cases and healthy controls. Individuals studied were selected from a clinical screening series, i.e., consecutive patients who underwent a colonoscopy after suspicion of colonic pathology. COL10A1 serum levels in 16 colon cancer patients and 16 healthy individuals were determined in duplicates (N=32).

### Statistical analysis

To assess the predictive ability of the biomarker, classification measures such as the area under the ROC curve (AUC), the classification error rate, sensitivity, specificity, positive predictive value, negative predictive value, or the Matthews correlation coefficient have been calculated.

To assess the prognostic significance value of the potential biomarker, Kaplan-Meier curves were performed to determine 10-year disease free survival for all patient groups. The patients were separated into two groups according to the biomarker mean levels. Univariate statistics using log rank test were calculated to assess the differences between survival curves and evaluate the prognostic significance of the biomarker.

### RESULTS

### Analysis in colon mucosa of COL10A1 mRNA expression

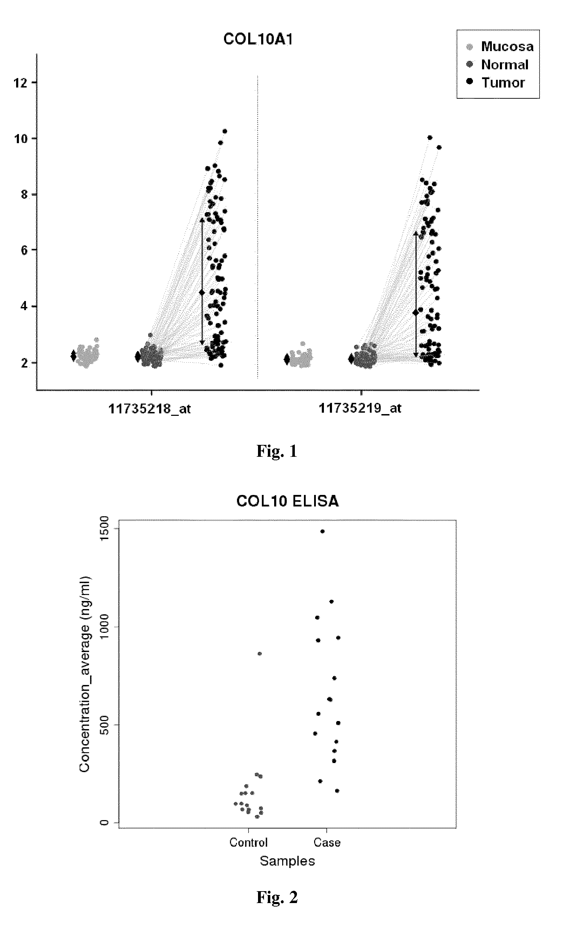

The analyses performed on COL10A1 expression data show significant differences between tissue types. The expression is very low in mucosa from healthy donors or normal mucosa from cases, but the tumor tissue shows very high expression values (Figure 1).

Differences in mean RNA expression levels between colon tumor, normal mucosa of patients and mucosa from healthy individuals are statistically significant (p<0.001). These differences are also observed with data from technical and biological validation (results not shown).

### Analysis in serum of COL10A1 protein

Serum levels of COL10A1 protein measured by ELISA were statistically significant different between cases and controls (p<0.0001, Figure 2). Moreover, a good correlation between duplicates has been observed (Figure 3).

Differences in serum COL10A1 protein levels are not due to differences in the stages of colon cancer cases, since stage II tumors already shows high levels of the candidate biomarker (Figure 4).

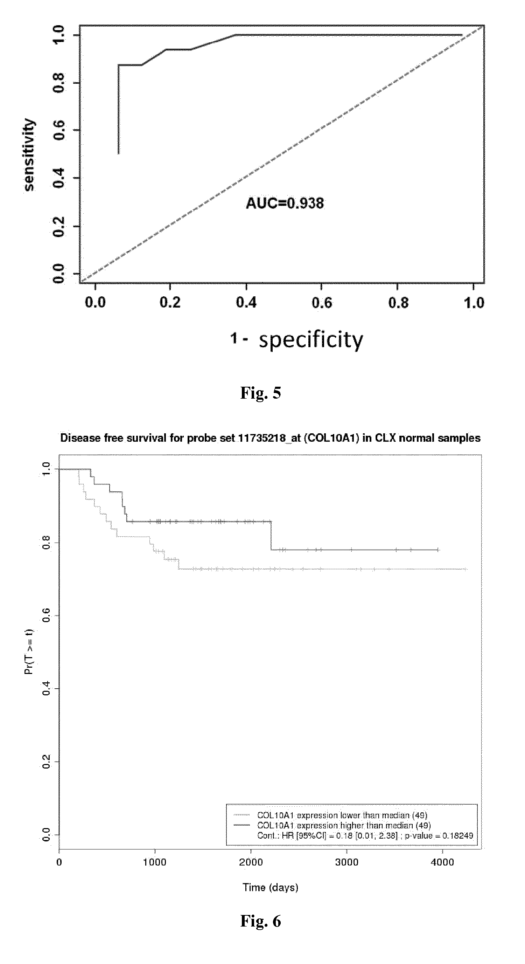

In this first test, the candidate biomarker show high values of sensibility and specificity, and as it has been shown in Figure 5, the AUC is 0.938.

### Survival analysis

Preliminary results show that RNA expression levels in colon cancer patients of the candidate biomarker have a small, non-significant, prognostic value (Figure 6). However, since the presence of COL10A1 in serum is indicative of colorectal tumor, this biomarker may be useful to monitor therapy response.

## Claims

1. An *in vitro* method for diagnosing colorectal cancer in a subject comprising determining the level of COL10A1 protein in a biofluid of said subject, wherein an increased COL10A1 protein level is indicative of colorectal cancer.

2. The method of claim 1, wherein said colorectal cancer is stage 0, stage I, stage II, stage III and/or stage IV colorectal cancer.

3. The method of any of claims 1 to 2, wherein the subject is a subject to which at least one colorectal cancer risk factor applies.

4. The method comprising to any one of claims 1 to 3, wherein the increase is determined by comparison to a reference value.

5. The method according to claim 4, wherein the reference value is the average or median level of COL10A1 protein in biofluid in a group of subjects not having colorectal cancer.

6. The method of any one of claims 1 to, 5 further comprising the determination of the levels of one or more biomarkers selected from the group consisting of CEA, TCN, SULT2B1, ALDOB, COL11A1, PI3, CCL20, MTHFD11, IL-1b, SRPX2, SLC04A1, TESC, and/or IL-23a wherein an increase in the levels or one or more of said biomarkers is indicative of colorectal cancer.

7. The method of any one of claims 1 to 6, further comprising one or more diagnostic means selected from the group consisting of digital rectal exam, fecal occult blood test, sigmoidoscopy, colonoscopy, colonography and an imaging test.

8. A method for monitoring the effect of a therapy in at least one patient suffering from colorectal cancer and being treated with said therapy, comprising
(i) determining the level of COL10A1 protein in a biofluid of said patient, and
(ii) comparing said level of COL10A1 protein to at least one level of COL10A1 protein in said biofluid of said patient determined at the start of the therapy and/or determined previously during said therapy,
wherein a stagnation and/or a decrease of the level of COL10A1 protein in said patient indicates a positive effect of said therapy.

9. A method for the identification of a therapy for colorectal cancer comprising
(i) determining the level of COL10A1 protein in a biofluid of at least one patient suffering colorectal cancer and which is being treated with a candidate therapy, and
(ii) comparing the level of COL10A1 protein determined in step (i) to at least a reference value for said COL10A1 protein levels wherein a stagnation and/or a decrease of the level of COL10A1 protein in the biofluid with respect to the reference value indicates that the candidate therapy is useful for treating colorectal cancer
wherein said reference value is selected from the group consisting of the COL10A1 protein expression levels in the biofluid of said patient determined at the start of the therapy, the COL10A1 protein expression levels in the biofluid of said patient determined previously during said therapy and the level of COL10A1 protein in the biofluid of at least one control patient not treated with said candidate therapy.

10. The method of claim 8 or 9, further comprising the determination of the levels of one or more biomarkers selected from the group consisting of CEA, TCN, SULT2B1, ALDOB, COL11A1, PI3, CCL20, MTHFD11, IL-1b, SRPX2, SLC04A1, TESC, and/or IL-23a
wherein an stagnation and/or a decrease of the level of one or more of said biomarkers is indicative of a positive effect of said therapy, or
wherein a stagnation and/or a decrease of the level of one or more of said biomarkers in the biofluid with respect to the reference value indicates that the candidate therapy is useful for treating colorectal cancer.

11. The method of claims 8, 9 or 10, further comprising one or more diagnostic means selected from the group consisting of digital rectal exam, fecal occult blood test, sigmoidoscopy, colonoscopy, colonography and an imaging test.

12. The method of to any of claims 1 to 11, wherein said biofluid is blood, plasma or serum, and preferably is serum.

13. Use of a COL10A1 protein antibody for the diagnosis of colorectal cancer, for monitoring the effect of a therapy in a patient suffering from colorectal cancer or for the identification of a therapy for colorectal cancer.

14. A kit comprising means for carrying out the method of any one of claims 1 to 12 or for the use according to claim 13.

15. The kit of claim 14, wherein said kit comprises a COL10A1 antibody and one or more antibodies selected from the group consisting of CEA, TCN, SULT2B1, ALDOB, COL11A1, PI3, CCL20, MTHFD11, IL-1b, SRPX2, SLC04A1, TESC, and/or IL-23a antibody.
